# EUROPEAN PATENT APPLICATION

(11) **EP 4 338 779 A1**
(43) Date of publication of application: **20.03.2024**
(21) Application number: 22196032.1
(22) Date of filing: 16.09.2022
(51) Int. Cl.: A61M 25/00, B29C 45/00, B29C 64/00, B33Y 80/00, A61M 25/01

(54) **GUIDE WIRE EXIT PORT, CATHETER AND MANUFACTURING METHODS FOR SAME**

(71) Applicant: BIOTRONIK AG, 8180 Bülach (CH)
(72) Inventor: Wintsch, Daniel, 8050 Zürich (CH); Klaus, Sebastian, 8424 Embrach (CH); Gimmel, Christian, 8408 Winterthur (CH)
(74) Representative: Biotronik Corporate Services SE

(57) **Abstract**

The invention refers to a manufacturing method for a guide wire exit port (1, 101, 201, 301), wherein the guide wire exit port is adapted to receive a hypotube (35) at a proximal end and an outer tubing (36) with an inner tubing (37) at a distal end, wherein the guide wire exit port is manufactured by additive manufacturing being a single guide wire exit port member (301) or first consisting of at least two guide wire exit port members and/or by injection molding first consisting of at least two guide wire exit port members (21, 22, 121, 122, 221, 222), wherein the at least two guide wire exit port members are pressure-tightly connected thereafter. The invention further refers to a manufacturing method for a catheter (30, 330) comprising the guide wire exit port, the respective guide wire exit port, and the respective catheter.

## Description

The present invention relates to a guide wire exit port, a catheter as well as to a manufacturing method for such guide wire exit port and for a catheter having such guide wire exit port.

Rapid-exchange (RX) or monorail catheters (sometimes also called fast-exchange or single operator exchange catheters) are catheters in which a guide wire is tracked over a distal portion of the catheter. In a portion proximal of the distal portion that tracks the guide wire the RX catheter and the guide wire are separate from one another. This allows the proximal portion of the RX catheter to be relatively simple having need of one less lumen than would be required for an over-the-wire catheter that tracks a guide wire over its entire length. An RX catheter is also advantageous because the catheter and the guide wire are more easily moved relative one another such as during a catheter exchange.

Manufacturing of present RX catheters involves welding or fusing several lengths of tubing together such that the distal portion of the RX catheter includes an additional lumen for receiving the guide wire. Further, manufacturing involves manually providing (e.g. drilling) an opening into the welded or fused tubing to allow the guide wire to be introduced into the guide wire lumen. Such manufacturing steps with regard to the guide wire exit port are complicated, cost and time intensive and quality deficiency prone. Additionally, automation of such process steps is hardly possible.

Document US 2005/0049552 A1 discloses a catheter and a method of making such catheter wherein a port joint for coupling a proximal member to one or more distal members is used, wherein the port joint includes a guide wire exit port and is attached to the proximal member and to one or more distal members, wherein the port joint is provided as a single piece by injection molding.

Accordingly, it is an objective of the present invention to provide an automatable manufacturing method of a guide wire exit port and of a catheter having a small outer diameter. Additionally, it is an objective of the present invention to realize radial assembly in the area of the guide wire exit port and high accuracy and dimensional stability of the joint.

The above problem is solved by a manufacturing method for a guide wire exit port having the features of claim 1, a manufacturing method for a catheter having the features of claim 12, a guide wire exit port having the features of claim 7 and a catheter having the features of claim 15.

In particular, the above problem is solved by a manufacturing method for a guide wire exit port. The guide wire exit port is adapted to receive a hypotube at a proximal end and an outer tubing with an inner tubing at a distal end. The guide wire exit port is manufactured by additive manufacturing and/or by injection molding.

The guide wire exit port may be manufactured by injection molding at least two guide wire exit port members, wherein the at least two guide wire exit port members are connected thereafter.

The guide wire exit port may be manufactured by additive manufacturing a single guide wire exit port member or at least two guide wire exit port members, wherein the at least two guide wire exit port members are connected thereafter.

The guide wire exit port manufactured by above method may be formed as an elongated part extending in a longitudinal direction having a proximal end and a distal end. The longitudinal direction is essentially parallel or identical to the longitudinal axis of the hypotube and the inner and outer tubing. As described below in detail, if mounted in the catheter the guide wire exit port is used to be accommodated between the hypotube and the outer tubing with the inner tubing (e.g. the inner tubing is accommodated by and runs within the inner lumen of the outer tubing), fixed at the hypotube, the outer tubing and, if applicable, also at the inner tubing and comprises an opening for introduction of the guide wire into the guide wire lumen (usually within the inner tubing). For fixing the hypotube and the outer tubing with the inner tubing to the guide wire exit port, it comprises a cylindrical opening at its proximal end and a cylindrical opening at its distal end, wherein each opening is connected to the other one and to the opening for the guide wire. The guide wire exit port is used to create a smooth transition between the hypotube forming the proximal section and the distal section of the catheter where the guide wire runs within the guide wire lumen. For that, the guide wire exit port is adapted to receive the hypotube at its proximal end (so-called proximal section), for example by a hollow cylindrical proximal section. In one embodiment, the inner diameter of the hollow cylindrical proximal section is adapted to the outer diameter of the hypotube so that the hypotube can be inserted within the hollow cylindrical proximal section such that the outer surface of the hypotube contacts the inner surface of the proximal section of the guide wire exit port at least over part of its length. Further, in one embodiment, the guide wire exit port is adapted to receive the outer tubing at its distal end (so-called distal section) and therefore may comprise, for example, a hollow cylindrical distal section. In one embodiment, the inner diameter of the hollow cylindrical distal section is adapted to the outer diameter of the outer tubing so that the outer tubing can be inserted within the hollow cylindrical distal section such that the outer surface of the of the outer tubing contacts the inner surface of the distal section of the guide wire exit port at least over part of its length. The inner tubing may be accommodated within the lumen of the outer tubing. Further, the guide wire exit port may comprise a center section having a through-going opening in a wall of the guide wire exit port that is adapted with regard to its size such that it may receive the guide wire and lead it into the distal section of the catheter, e.g. the inner lumen of the inner tubing. Accordingly, the diameter of the opening is greater than the outer diameter of the guide wire. Further, the center section may be adapted to receive the inner tubing at its distal end and may be formed as a hollow cylinder at its distal end. The axis of this hollow cylinder of the center section of the guide wire exit port may be inclined with regard to the axis of the hollow cylinder of the proximal section and the hollow cylinder of the distal section of the guide wire exit port in order to provide a smooth transition of the guide wire running within the inner tubing.

In one embodiment, the guide wire exit port may be manufactured by additive manufacturing (three dimensional (3D) printing) as a single guide wire exit port member or as at least two guide wire exit port members, wherein the at least two guide wire exit port members are pressure-tightly connected thereafter. For example, exactly two members may be produced by additive manufacturing, e.g. two half shell members of the guide wire exit port.

Additive manufacturing (also called generative manufacturing) refers to a variety of manufacturing processes in which material is deposited, joined or solidified under computer control to create a three-dimensional object, with material being added together (e.g. fused together), typically layer by layer. For example, for each layer, a material cohesion is created by melting or chemical curing processes before the next layer is applied. The advantage of additive manufacturing is that the complex (inner) geometric form of a guide wire exit port is individually producible based on CAD data or 3D digital data (3D model). Accordingly, guide wire exit ports for different catheter types may be produced within one production facility. In one embodiment, a plurality of guide wire exit ports as single guide wire exit port members or at least two guide wire exit port members may be produced in parallel within one additive manufacturing facility. Such manufacturing method allows for a high degree of automation. Additionally, additive manufacturing methods allow to produce complex geometric forms with small dimensions so that the outer diameter of a guide wire exit port produced by additive manufacturing may be small.

In one embodiment, a polymer material and/or a metal material may be used for production of the guide wire exit port or at least one of its at least two guide wire exit port members by additive manufacturing. For example, the guide wire exit port may comprise at least one material of the group comprising thermoplastics (such as polycarbonates), thermoplastic elastomers (such as polyamides like polyamide 12), stainless steel, CoCr alloys, and nickeltitanium alloys (e.g. Nitinol). In one embodiment, for production of a metal guide wire exit port or its at least two guide wire exit port members selective laser melting, selective electron beam melting or metal binder jetting may be used.

In one embodiment, the single guide wire exit port member of the guide wire exit port or the at least two guide wire exit port members of the guide wire exit port is/are post-processed after additive manufacturing, for example with regard to its/their outer surface and/or its/their inner surface, e.g. by microjetting, micro machining processing (MMP), ultrasound processing (with or without abrasive paste), trowalizing (vibratory, slide or smooth grinding, barrel finishing), hot acid etching, burst deburring (oxidizing of particles from the surface by temperature peaks, for example by ignition of a methane oxygen mixture). MMP is a mechanical chemical process where a carrier medium loaded with abrasive particles flows through the treated object. In one embodiment, the inner surface and the outer surface of the single guide wire exit port member guide wire exit port or the at least two guide wire exit port members of the guide wire exit port may be post-processed using different processes or the same processes and/or a plurality of members may be post-processed in parallel (i.e. at the same time).

In one embodiment, the guide wire exit port may be manufactured by injection molding first consisting of at least two guide wire exit port members, wherein the at least two guide wire exit port members are pressure-tightly connected thereafter. For example, the guide wire exit port may be manufactured by injection molding first consisting by exactly two members, for example each one forming one half shell member of the guide wire exit port. For example, each half shell member extends over the full length of the guide wire exit port in its longitudinal direction but comprises only one fraction, e.g. one half, of the full circumference of the guide wire exit port. In another example, one "half" shell member may comprise another fraction of the full circumference, e.g. one may comprise 65% of the full circumference of the guide wire exit port and the other "half" shell member may comprise 35% of the full circumference of the guide wire exit port. In one example, the form of the guide wire exit port may be such that it consists of two inverse halves forming the two half shell members. The advantage is, that the two half shell members of the guide wire exit port are easy to produce by injection molding in a cost-effective way and with small dimensions. Additionally, using the two half shells prior to connection to each other, the hypotube and/or the inner tubing and/or the outer tubing may easily be accommodated within one of the two half shells and thereby allow radial mounting and automation of the catheter production with regard to the guide wire exit port area. Additionally, only little or no post-processing of the at least two guide wire exit port members of the guide wire exit port may be necessary. In contrast to additive manufacturing, where workpiece surfaces are generally rougher and/or ridged due to the build-up of additive layers and thus require post-process smoothing and/or finishing, injection molding results in an essentially uniform and smooth finish with minimal surface irregularities, because the material is injected into the mold as a single layer precisely defined by the size, volume, shape and/or contour of said mold.

The at least two guide wire exit port members of the guide wire exit port may comprise, for example, at least one material of the group comprising thermoplastics, such as polycarbonates, and thermoplastic elastomers, such as polyamide 12, and related plastics.

In one embodiment, the injection molding of the at least two guide wire exit port members of the guide wire exit port is provided using film gate or pin gate or any other suitable gate design. If pin gate is used, the gate may be directly removed when the member is removed from the mold. If film gate is used, the mold filling may be provided more uniformly. Additionally, the gate may be used for handling of the respective member. If the film gate is removed from the member, distortion of the respective member is reduced. With regard to a pin gate or a film gate - both gate types may be removed, for example, by cutting off or breaking off the gate from the respective member.

In one embodiment with regard to manufacturing the at least two guide wire exit port members by additive manufacturing or injection molding, the at least two guide wire exit port members may be manufactured first completely separately or connected by a small web, e.g. a film gate. In the latter embodiment, each two of the at least two guide wire exit port members may be connected by a small web which eases handling. The web may be cut off or broken off later similarly to a film gate, e.g. after pressure-tightly connection of the at least two guide wire exit port members thereby forming the guide wire exit port. The web may be used as a positioning aid as explained below.

In one embodiment, the at least two guide wire exit port members is/are post-processed after injection molding, for example with regard to its/their outer surface and/or its/their inner surface. For example, the above-mentioned post-processing methods may be used, as well.

With respect to injection molding, other post-processing methods like degating (removing pin or film gates or any other suitable gate designs), deflashing or deburring (e.g. removing excess materials such as burrs, excess (thermo-)plastic material or any resin bleed), cleaning (e.g. removing processing aids or residues like dirt, grease or other contaminants that would interfere with downstream processing or manufacturing steps) and/or decorating (e.g. painting, plating, vacuum metallizing, pad printing, hot stamping, silk screening, fill and wipe) may be used.

In one embodiment, the at least two guide wire exit port members of the guide wire exit port which are produced by additive manufacturing or injection molding as explained above may be (pressure-tightly) connected, for example, by laser welding or fusing. For example, neighboring members of the at least two guide wire exit port members are accommodated such that they contact each other at a connection area and are then interconnected/joined by welding or fusing thereby forming a substance-to-substance-bond in the form of a weld or a fused seam/joint. Pressure-tight or pressure-resistant connection means that such connection resists strains like, for example, mechanical load up to at least 30 atm. Alternative methods to connect the at least two guide wire exit port members are any plastics joining method including but not limited to ultrasonic welding, adhesive bonding, glueing or any other suitable physical or chemical bonding or fusing method as well as force-fit and form-fit connections. Laser welding or fusing may be easily automated. In one embodiment, the at least two guide wire exit port members, e.g. the two half shell members, may be placed within a carrier that fixes the at least two guide wire exit port members with respect to each other. Such carrier may provide at least one suitable recess which allows exact application of the laser beam at the area(s) to be welded. Such carrier may be rotatable or otherwise movable to provide the laser beam at the area(s) to be welded at the surface or an inner region of the at least two guide wire exit port members. Alternatively or additionally, the laser beam may be movable.

Laser welding or fusing may be provided by a laser or a heating element which is applied to the portion of the at least two guide wire exit port members to be connected. For laser welding, for example, a Raman-laser or any other suitable laser having a wavelength emission in the near-infrared region of the electromagnetic spectrum from 780 nm to 2500 nm, preferably from 1700 nm to 1800 nm, may be used. For example, if the guide wire exit port is manufactured from two half shell members, both extending in longitudinal direction, a laser or heating element may be applied to an area extending longitudinally along the full length of both members where the two half shell members adjoin to connect these members.

In one embodiment, the at least two guide wire exit port members are connected using at least one positioning aid that is provided at each one of the at least two guide wire exit port members of the guide wire exit port. The positioning aid is used to arrange the at least two guide wire exit port members in a pre-defined position prior pressure-tight connection of the at least two guide wire exit port members, e.g. the two half shells, of the guide wire exit port. For example, two opposing positioning aids of two members to be connected may realize a form fit, for example one positioning aid is a protruding pin and another positioning aid is a corresponding recess or opening, each one located, for example, at a flap of the respective member which may be provided at a side surface. In one embodiment the at least one positioning aid is removed after finishing the pressure-tight connection of the at least two guide wire exit port members of the guide wire exit port.

The above problem is also solved by a manufacturing method for a catheter, which may be an RX catheter, wherein the method comprises the steps:
Providing a hypotube, an outer tubing with an inner tubing and a guide wire exit port or at least two guide wire exit port members of a guide wire exit port, wherein the guide wire exit port is manufactured using the method steps defined above and
Attaching the hypotube, the outer tubing and the inner tubing to the guide wire exit port or to one of its members. The attachment of the hypotube, the outer tubing and the inner tubing to the guide wire exit port or to one of its members is described below in detail.

In one embodiment, the manufacturing method for the catheter additionally comprises the steps:
Inserting the hypotube into a proximal section of the guide wire exit port or of one of its members,
Inserting the outer tubing and the inner tubing into a distal section of the guide wire exit port or of one of its members, wherein in case the guide wire exit port is manufactured from at least two guide wire exit port members the hypotube and/or the inner tubing and/or the outer tubing is/are inserted into the guide wire exit port after the at least two guide wire exit port members of the guide wire exit port are pressure-tightly connected or into one of at least two guide wire exit port members of the guide wire exit port prior the at least two guide wire exit port members are pressure-tightly connected.

For example, the above manufacturing method for a catheter includes an embodiment where the guide wire exit port is one single guide wire exit port member manufactured by additive manufacturing as explained above and an embodiment where the guide wire exit port is assembled from at least two separate members (manufactured by additive manufacturing or injection molding) which are pressure-tightly connected prior inserting the hypotube into a proximal section of the guide wire exit port and/or inserting the outer tubing and/or the inner tubing into a distal section of the guide wire exit port. In these cases, the hypotube and/or the outer tubing and/or the inner tubing may be inserted by moving/shifting it into longitudinal direction into a respective longitudinally extending opening of the guide wire exit port. With regard to the outer tubing and the inner tubing, they may be moved together in longitudinal direction at least over a part of the moving distance. Further, an embodiment is included where the hypotube and/or the outer tubing and/or the inner tubing is inserted into a distal and proximal section of the guide wire exit port, respectively, prior the at least two guide wire exit port members of the guide wire exit port are pressure-tightly connected. As in these embodiments the guide wire exit port may be not fully closed, the hypotube and/or the outer tubing and/or the inner tubing may be radially and/or axially moved/put into the pre-determined recess of the respective member of the guide wire exit port.

In one embodiment, attaching the hypotube, the inner tubing and/or the outer tubing is provided by additive manufacturing or injection molding and/or attaching the hypotube, the outer tubing and/or the inner tubing to the guide wire exit port or to one of its members is provided by laser welding or by fusing. This means that each one of the hypotube, the inner tubing and the outer tubing may be either attached to the guide wire exit port or one of its at least two guide wire exit port members during additive manufacturing or injection molding or in a separate step by laser welding or by fusing. If the attachment is provided during additive manufacturing or injection molding, the guide wire exit port or one of its members is manufactured around the respective tube/tubing so that they/their material are/is fixed to each other at the same time, for example by an adhesive or force-fit joint.

In one embodiment, the attachment of the hypotube, the outer tubing and/or the inner tubing to the guide wire exit port or to one of its members is provided by laser welding or by fusing in a pre-defined area of the hypotube, the outer tubing and/or the inner tubing which is accommodated adjacent to the guide wire exit port or one of its members before that. For laser welding, for example, a Raman-Laser having a wavelength of 1700 nm may be used. In this case, the material of the guide wire exit port or its members may be at least partially transparent to the used electromagnetic radiation of the laser or the heat energy produced by fusing to realize the connection between the hypotube and the tubing to an inner surface of the guide wire exit port or one of its members.

The above problem is also solved by a guide wire exit port manufactured using the manufacturing steps defined above.

The wall thicknesses of the guide wire exit port may be between 0.05-0.2 mm, preferably between 0.1 mm and 0.15 mm.

In one embodiment, the guide wire exit port comprises a distal section, a proximal section and a center section located between the distal section and the proximal section, wherein the center section comprises the opening for the guide wire, as explained above. In one embodiment, the center section has a wall thickness that is at least 30% greater, preferably at least 50% greater, than a wall thickness of the distal section and/or a wall thickness of the proximal section to absorb radial forces due to introduction of the guide wire through the respective opening of the center section. For example, the wall thickness in the center section is 0.15 mm and the wall thickness in the distal section and/or the proximal section is 0.1 mm. As indicated above, the proximal section may be a hollow cylindrical section adapted to receive and to be attached to the hypotube. Additionally, the distal section may be a hollow cylindrical section adapted to receive and to be attached to the outer tubing and/or a part of the center section, for example the distal part of the center section may be a hollow cylindrical section adapted to receive and to be attached to the inner tubing.

In one embodiment, the length of the distal section and/or the proximal section in longitudinal direction may be between 2 mm and 10 mm. The length of the center section in longitudinal direction may be between 4 mm and 7 mm. The advantage of using such dimensions is that they lie in a range particularly suitable in terms of manufacturablity by injection molding and realizing pressure-resistant connections to distal and/or proximal portions of the catheter. In addition, the suggested dimensions also ensure that variations in bending stiffness are minimal and that the flexural behavior is only affected on a short section of the catheter.

In one embodiment, the distal section of the guide wire exit port has an outer diameter that is between 120% and 150% of an outer diameter of the proximal section. For example, the outer diameter of the distal section is 1.1 mm and the outer diameter of the proximal section is 0.85 mm. Therein, the outer diameter is defined as the diameter of the guide wire exit port with regard to its outer surface.

In one embodiment, the center section of the guide wire exit port comprises a web (also referred to as bar or bridge) that extends longitudinally from the outer wall of the proximal section distally into the center section and divides the inner lumen of the center section into two compartments along a portion of the center section having a length in longitudinal direction of at least 1.5 mm. This web may divide the inner lumen of the center section into one lumen that is adapted to connect the inner lumen of the hypotube accommodated within the proximal section and the lumen between the inner tubing and the outer tubing accommodated within the distal section after completion of the catheter and into another lumen that is adapted to receive the guide wire and to connect this lumen to the inner lumen of the inner tubing. Accordingly, another lumen may be connected to the opening for the guide wire, for example at its proximal end. At least one of the two compartments may be formed at least partially as a hollow cylinder, wherein the longitudinal axis of this hollow cylinder may be inclined by 45° and less, preferably by 30° and less, with regard to the longitudinal axis of the guide wire exit port or its distal section or its proximal section.

The above problem is also solved by a catheter comprising a hypotube, an outer tubing with an inner tubing and a guide wire exit port, wherein the catheter was manufactured using the manufacturing steps as described above. Such catheter comprises the above embodiments and has the above explained advantages.

The above problem is also solved by a catheter comprising a guide wire exit port as described above. Such catheter comprises the above embodiments and has the above explained advantages.

Further manufacturing steps of such catheters include manufacturing the distal portion of the catheter by assembling and bonding together inner tubing, outer tubing and balloon. Subsequently, the distal portion is connected to the hypotube via the guide wire exit port as described in the present invention. Then, the balloon is pleated and fixated on the distal portion of the catheter and the fully assembled catheter is finally sterilized and wrapped into a packaging comprising an inner pouch and outer box.

The various features and advantages of the present invention may be more readily understood with reference to the following detailed description and the embodiments shown in the drawings. Herein schematically and exemplarily,
- Fig. 1: depicts a first embodiment of a guide wire exit port in a side view;
- Fig. 2: shows the guide wire exit port of Fig. 1 in a top view;
- Fig. 3: depicts a first half shell of the guide wire exit port of Fig. 1 in a side view (view along the arrows A-A shown in Fig. 2);
- Fig. 4: depicts a second half shell of the guide wire exit port of Fig. 1 in a side view (view along the arrows B-B shown in Fig. 2);
- Fig. 5: shows the guide wire exit port of Fig. 1 in a view shown in Fig. 4 by the arrows D-D;
- Fig. 6: shows the guide wire exit port of Fig. 1 in a cross-section marked in Fig. 4 by the arrows E-E;
- Fig. 7: shows the guide wire exit port of Fig. 1 in a cross-section marked in Fig. 4 by the arrows F-F;
- Fig. 8: shows the guide wire exit port of Fig. 1 in a view shown in Fig. 4 by the arrows G-G;
- Fig. 9: depicts a first embodiment of a catheter in the area of a second embodiment of a guide wire exit port in a perspective side view;
- Fig. 10: depicts the catheter of Fig. 9 in a side view;
- Fig. 11: shows the catheter of Fig. 9 in a top view;
- Fig. 12: depicts a longitudinal section of the catheter of Fig. 9 showing a first half shell of the guide wire exit port of Fig. 9 in a side view;
- Fig. 13: depicts a longitudinal section of the catheter of Fig. 9 showing a second half shell of the guide wire exit port of Fig. 9 in a side view;
- Fig. 14-19: shows manufacturing steps of the catheter of Fig. 9 with a carrier and a first half shell the embodiment of a guide wire exit port of Fig. 9 in a top view (Fig. 14 to 17), in a side view (Fig. 18) and in a perspective top view (Fig. 19);
- Fig. 20: shows an embodiment of a full catheter in a side view;
- Fig. 21: depicts an example of an injection molded half shell of a guide wire exit port with a pin gate in a perspective side view;
- Fig. 22: depicts another example of an injection molded half shell of a guide wire exit port with a film gate in a perspective side view;
- Fig. 23-25: depicts another example of injection molded half shells of a guide wire exit port with positioning aids and the connected half shells (Fig. 25), each in a perspective side view;
- Fig. 26-27: shows the example of Fig. 25 within a carrier in a perspective side view;
- Fig. 28: depicts the example of Fig. 25 in a front view with a cutting tool;
- Fig. 29-34: shows manufacturing steps of another embodiment of the guide wire exit port in a perspective side view with a carrier depicted partially transparent;
- Fig. 35: shows a fourth embodiment of a catheter with an example of a guide wire exit port in a partly longitudinal section;
- Fig. 36: depicts a further embodiment of a guide wire exit port in a perspective side view; and
- Fig. 37: shows a flowchart of one embodiment of a manufacturing method for the guide wire exit port of Fig. 36
- Fig. 38: depicts a half shell of a guide wire exit port.

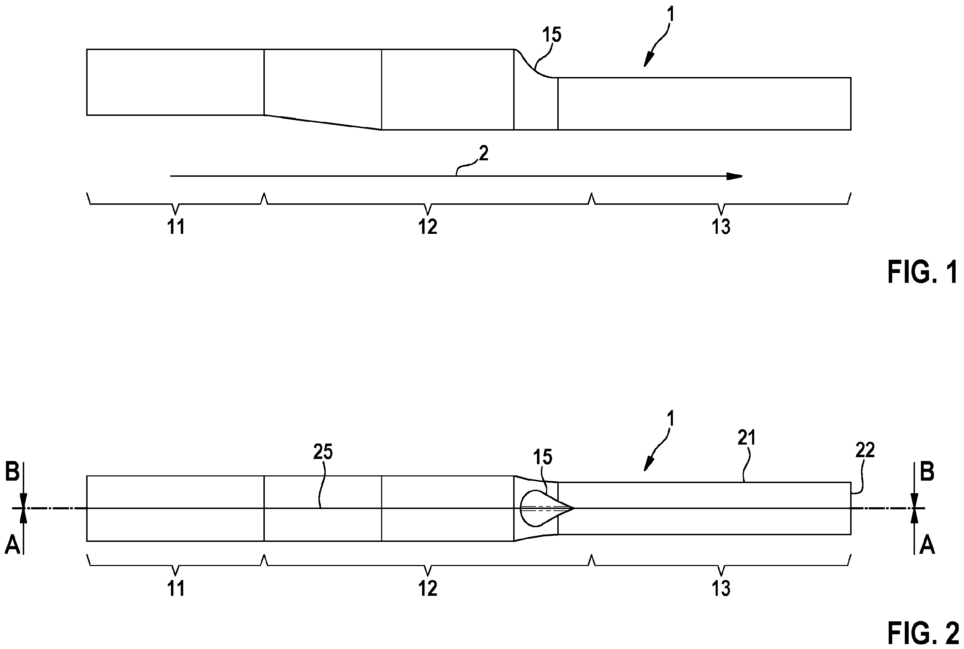

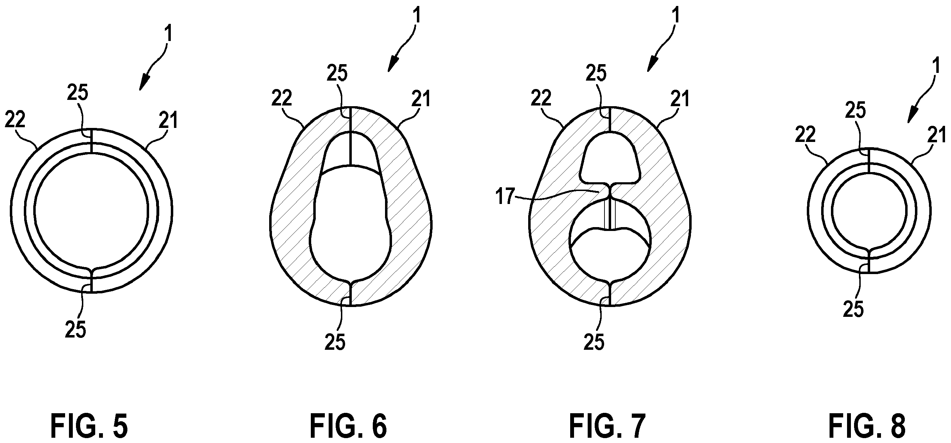

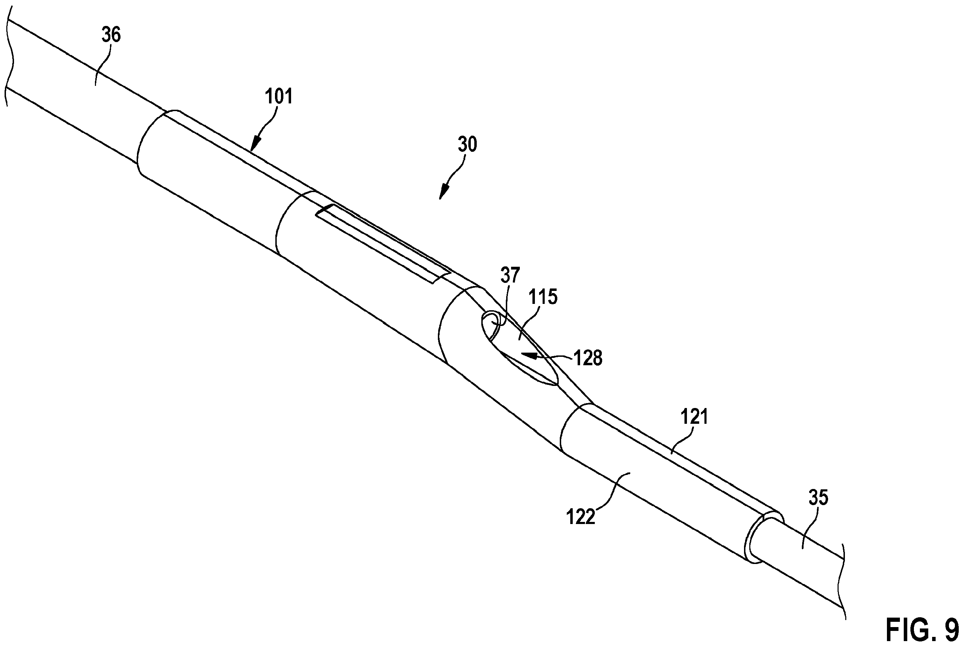

Fig. 1 to 8 show a first embodiment of an elongated guide wire exit port 1 having a hollow cylindrical distal section 11, a center section 12 and a hollow cylindrical proximal section 13. The center section 12 comprises an opening 15 for introduction of the guide wire and a web 17 dividing the lumen of the center section 12 into two compartments 18, 19. The accommodation of the hypotube / inner and outer tubing within the sections 11, 12, 13 after manufacturing of a catheter using the guide wire exit port 1 is similar to and described with regard to the second embodiment of a guide wire exit port shown in Fig. 9 to 13.

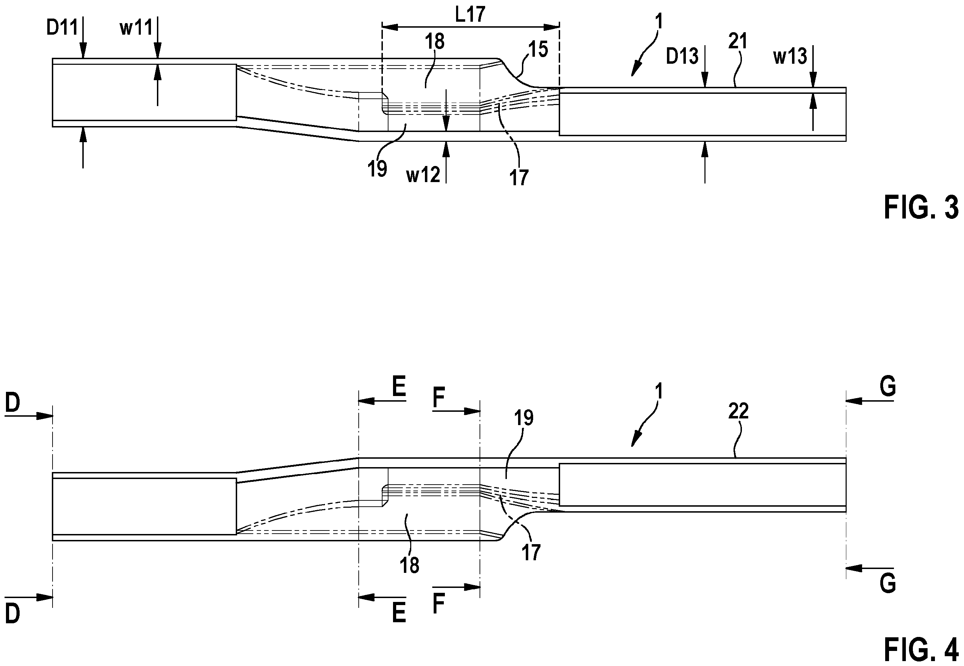

The guide wire exit port 1 is manufactured, for example, such that the two half shells shown in Fig. 3 and 4 are first produced separately by injection molding. Then, the two half shells 21, 22 are accommodated such that the two shells 21, 22 are adjacent to one another in close contact and connected using laser welding (e.g. Raman-Laser at a wavelength of 1700 nm). For that, the laser tip runs along the contact area of both half shells in longitudinal direction thereby forming a weld 25. The longitudinal direction is marked in Fig. 1 by arrow 2.

The guide wire exit port 1 has a wall thickness of the distal section w11 and a wall thickness of the proximal section w13 of, for example, about 0.1 mm. The wall thickness of the center section w12 is, for example, about 0.15 mm (see Fig. 3). Further, for example, the outer diameter of the proximal section D13 is about 0.85 mm, whereas the outer diameter of the distal section D11 is about 1.1 mm. The length L17 of the longitudinally extending web 17 is, for example, about 2.25 mm. The web 17 extends from the side wall of the proximal section 13 distally into the center section 12. The first compartment 18 comprises the opening 15 for the guide wire at its proximal end.

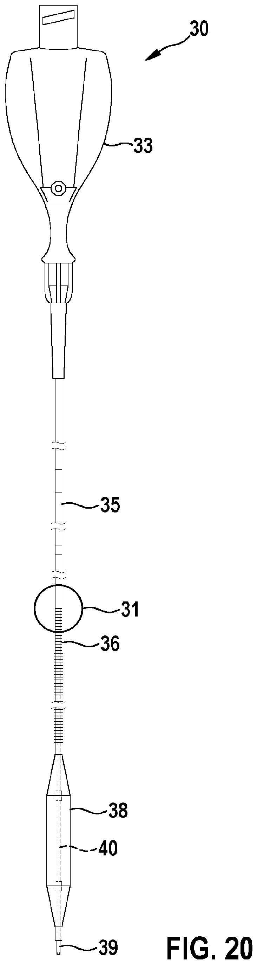

A first embodiment of a RX catheter 30 is partially shown in Fig. 9 to 13 comprising a second embodiment of a guide wire exit port 101. An example of a full catheter is provided in Fig. 20, where the section shown in Fig. 9 to 13 is highlighted by a circle 31. The catheter 30 comprises a handle 33 at its proximal end, a hypotube 35, an outer tubing 36, an inner tubing 37, a balloon 38, a distal tip 39 and a guide wire lumen 40 shown as a double dashed line. The guide wire runs outside and along the catheter in the region proximal from the area of circle 31. The guide wire lumen 40 mostly corresponds to the lumen within the inner tubing 37.

The elements of the guide wire exit port 101 correspond to the elements of the guide wire exit port 1, wherein the reference numbers of the second embodiment may be derived from the first embodiment by adding 100. It is therefore referred to the first embodiment of the guide wire exit port explained above.

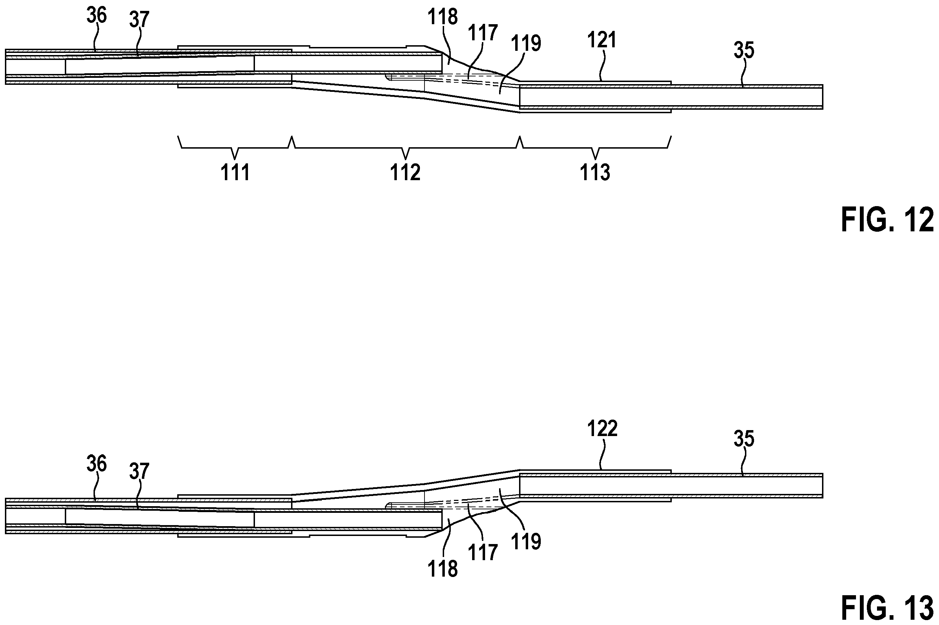

As best seen in Fig. 12 and 13 the outer tubing 36 is accommodated within the distal section 111 of the guide wire exit port 101 and the hypotube 35 within the proximal section 113 of the guide wire exit port 101. The inner tubing 37 runs within the inner lumen of the outer tubing 36 and extends from the proximal end of the outer tubing 36 into the center section 112 of the guide wire exit port 101, in particular into the first compartment 118 that accommodates the opening 115 for the guide wire. The introduction of the guide wire is symbolized by arrow 128 in Fig. 9. The second compartment 119 of the guide wire exit port 101 connects the inner lumen of hypotube 35 to the lumen between the outer tubing 36 and the inner tubing 37 that is used to inflate and deflate the balloon 38.

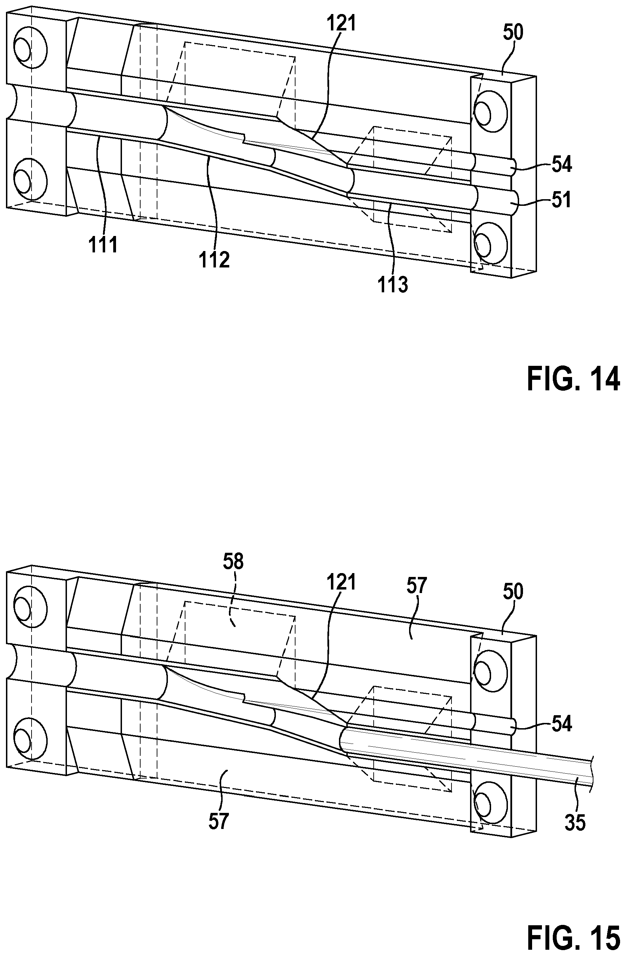

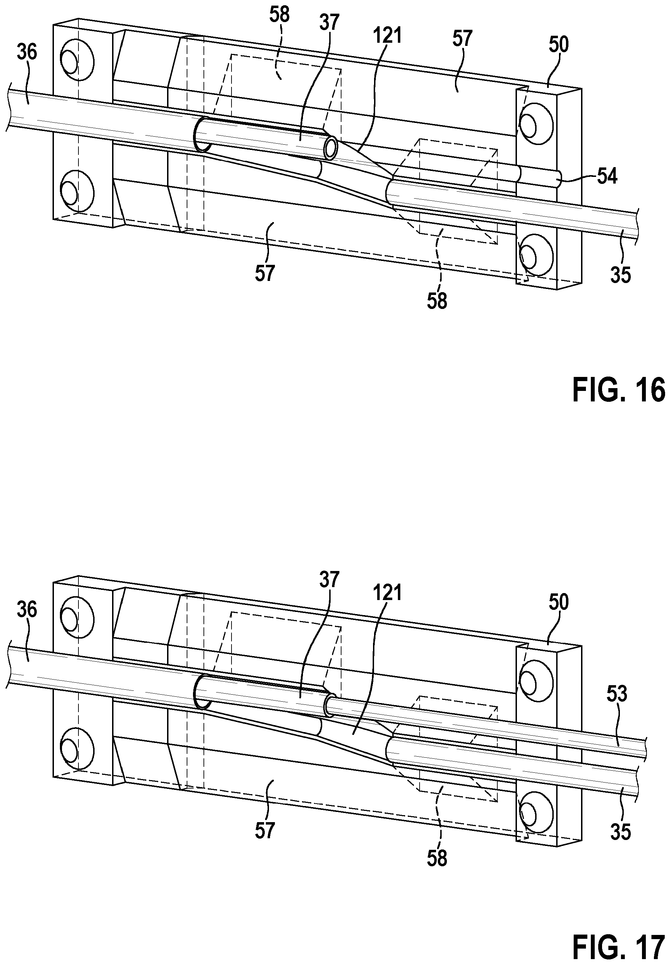

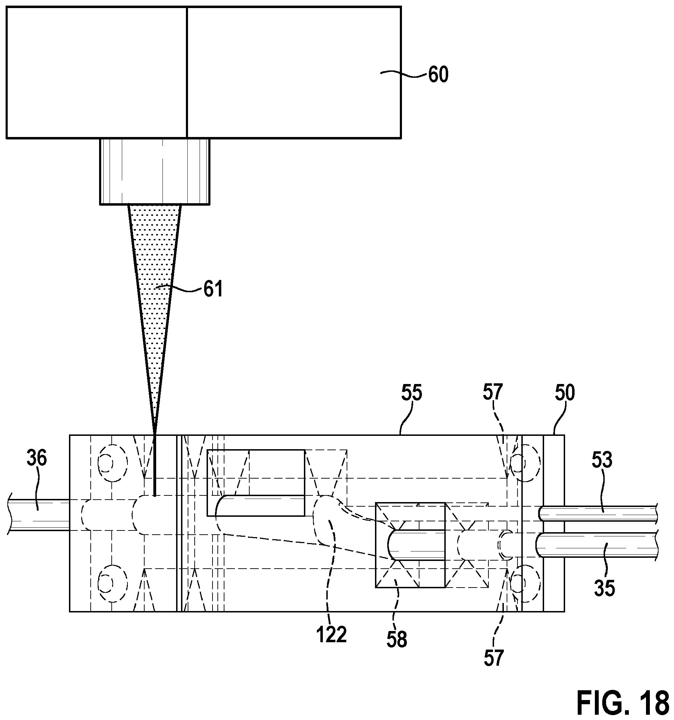

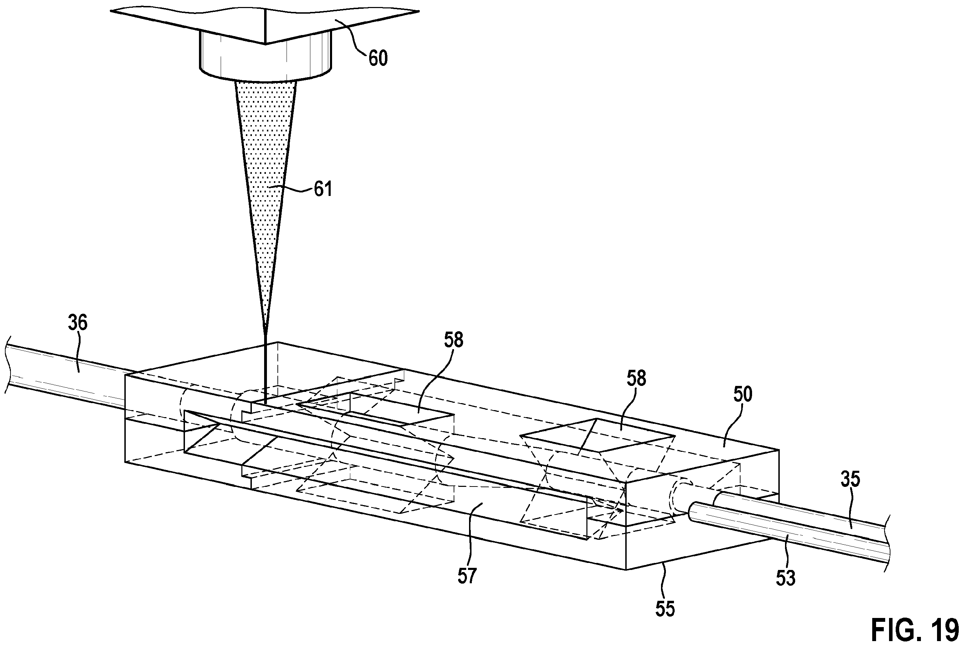

In the following, an example of a manufacturing method of the catheter 30 is explained with reference to the Fig. 14 to 19. In the first step, the first half 121 and the second half 122 of the guide wire exit port 101 are produced separately, for example by injection molding or additive manufacturing. In case of injection molding the first half 121 and the second half 122 may consist of, for example of a polymer without filling. Then, the first half 121 is accommodated within a recess 51 of a carrier 50 that corresponds to the outer form of the first half 121 (see Fig. 14). Then, the hypotube 35 is inserted into the proximal portion 113 of the first half 121 (see Fig. 15). Subsequently, the outer tubing 36 with the inner tubing 37 is inserted into the distal portion 111 of the first half 121 (Fig. 16), wherein the inner tubing 37 extends from the proximal end of the outer tubing 36 into the center section 112 of the first half 121. Afterwards, a retaining pin 53 is accommodated within the proximal end of the inner tubing 37 and within a corresponding recess 54 within the carrier 50 (Fig. 17). Then, the second half 122 is added such that it forms the inverse of the first half 121 of the guide wire exit port 101 and such that it is accommodated adjacent and in close contact to the first half 121. Additionally, the lid 55 of the carrier 50 is added thereby fixing the second half 122 with respect to the first half 121 (shown in Fig. 18 and 19).

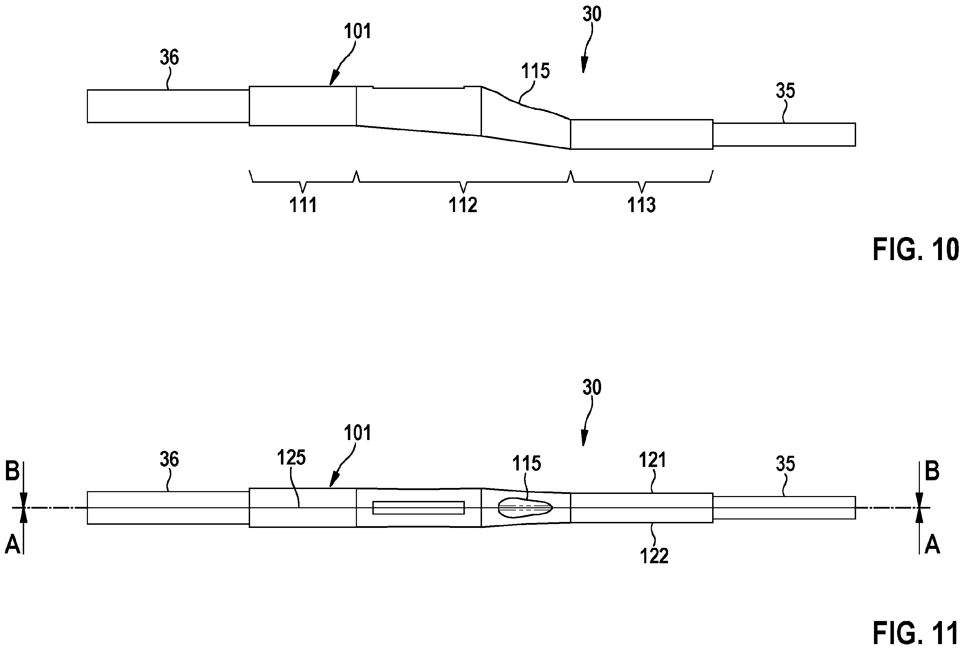

As one can derive from Fig. 18 and 19 the carrier 50 and its lid 55 may be moved and rotated such that a laser 60 (e.g. a Raman-Laser at a wavelength of 1700 nm) may weld the first half 121 and the second half 122 together such that these parts are pressure-tightly connected. For that, the carrier 50 and its lid provide respective recesses 57 so that the electromagnetic radiation 61 emitted by the laser (i.e. the laser light) directly reaches the area where the first half 121 and the second half 122 adjoin to each other. As a result, a longitudinally extending weld 125 is produced that runs along the guide wire exit port which may be best seen in Fig. 11. Additionally, along the side surfaces of the first half 121 and the second half 122 there are further recesses within the carrier 50 and the lid 55 which are marked by reference number 58 and which leave parts of the outer surface of the first half 121 and the second half 122, respectively, exposed. These recesses 58 are used for application of the electromagnetic radiation 61 emitted by the laser 60 to the surface of the first half 121 and the second half 122, respectively, to attach the outer tubing 36, the inner tubing 37 or the hypotube 35 to the guide wire exit port 101 in the respective region by welding. After finishing welding the carrier 50, the lid 55, and the retaining pin 53 may be removed. Now, the manufacturing of the catheter section as shown in Fig. 9 to 13 is completed. The other parts of the catheter 30 are provided and attached as known to the skilled person.

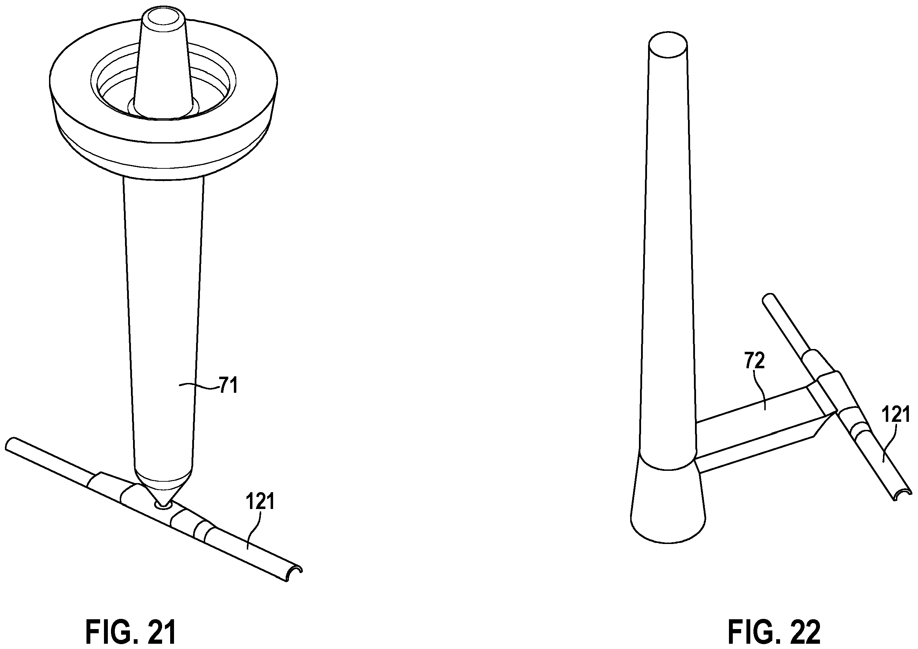

As shown in Fig. 21 and 22 the halves 121, 122 may comprise a pin gate 71 or a film gate 72 at the end of the injection molding step. These gates 71, 72 may be used for handling of the halves 121, 122 and may be removed by cutting prior connecting with the other half.

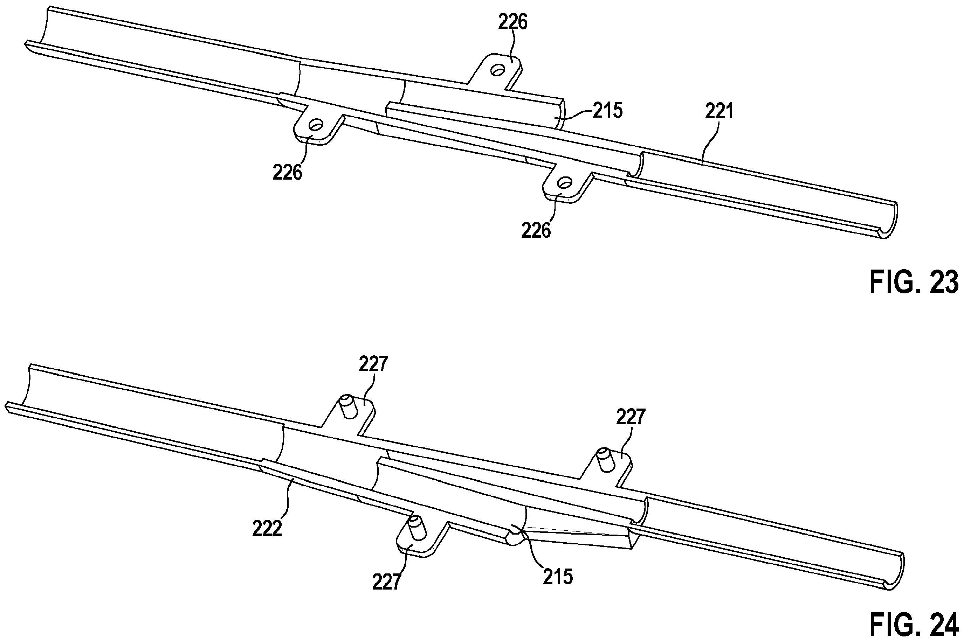

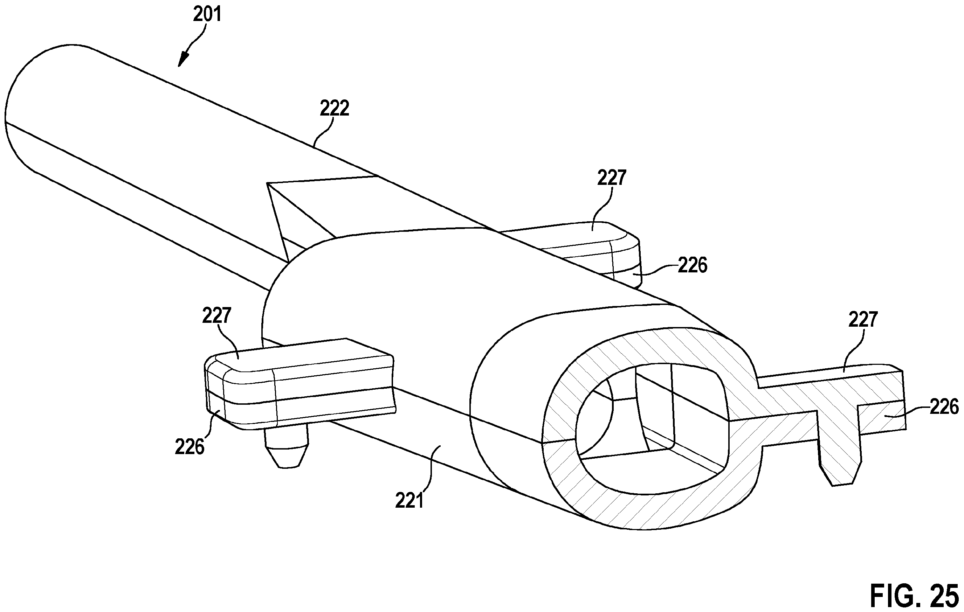

Fig. 23 to 28 shows a third embodiment of a guide wire exit port 201 with a first half 221 and a second half 222, wherein the first half 221 has three guiding aids in form of a flap 226 with a through-going opening and the second half has three guiding aids in form of a flap 227 with a protruding pin. Beside the flaps 226, 227 the members 221, 222 of the guide wire exit port 201 are similar to the first embodiment explained with regard to Fig. 1 to 8 above. The reference numbers of the members 221, 222 correspond to the reference numbers of the first embodiment by adding 200 and it is referred to the above explanation of the first embodiment of the guide wire exit port and its members.

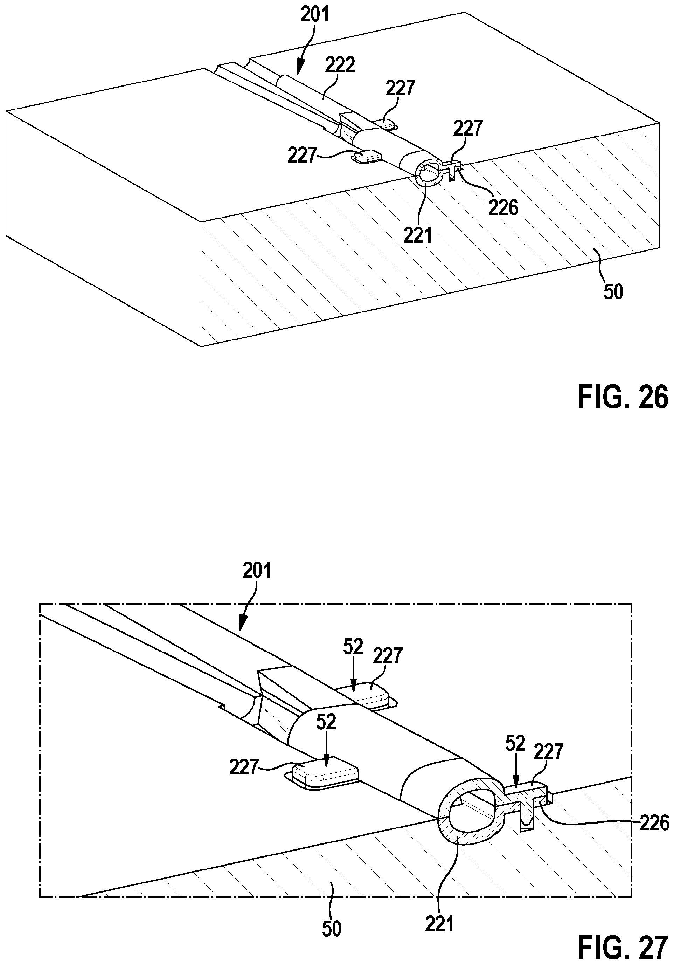

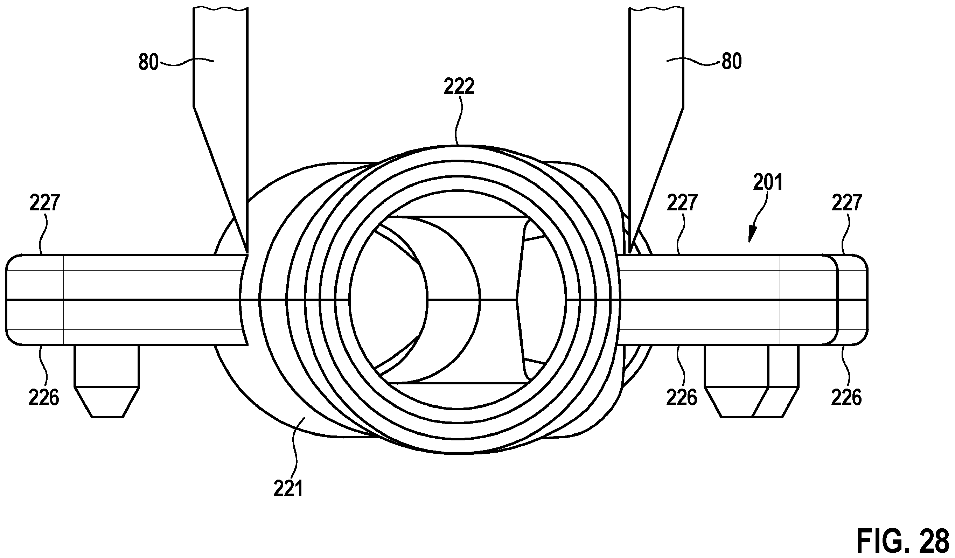

The flaps 226, 227 are located at the center section of the respective half 221, 222 extending radially from the outer surface of each half 221, 222 with regard to the longitudinal direction 2 (see Fig. 23, 24). As one can derive from Fig. 26 these positioning aids are used to position the first half 221 and the second half 222 as pre-defined to each other prior welding by introducing the respective pin of the flaps 227 into the respective through-going opening of the flaps 226 using a force that is perpendicular to the surface of the carrier 50 or of the flap 226 (see arrows 52 in Fig. 27). After welding the first half 221 and the second half 222 together, the flaps 226, 227 may be removed by cutting using a cutting tool 80 as shown in Fig. 28.

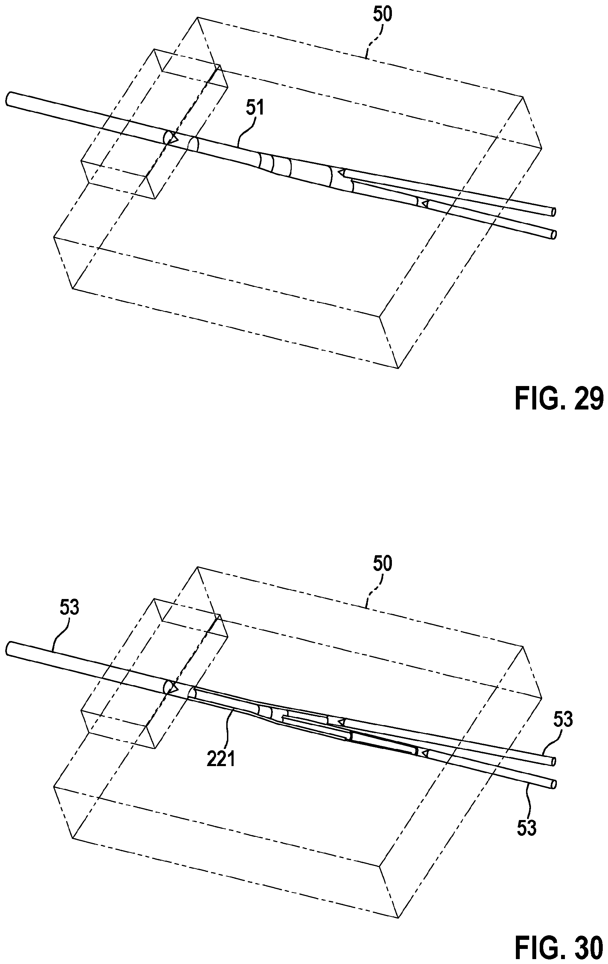

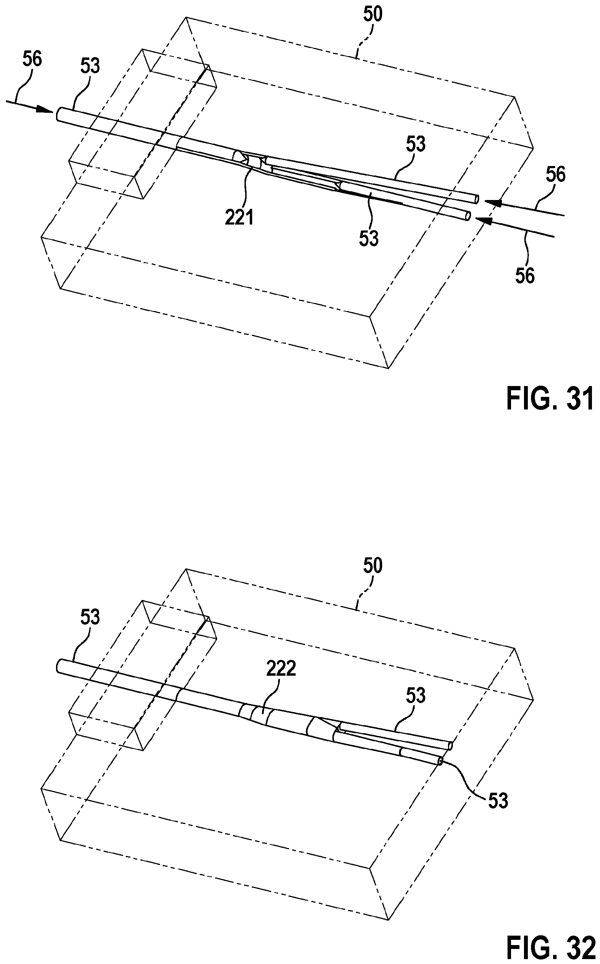

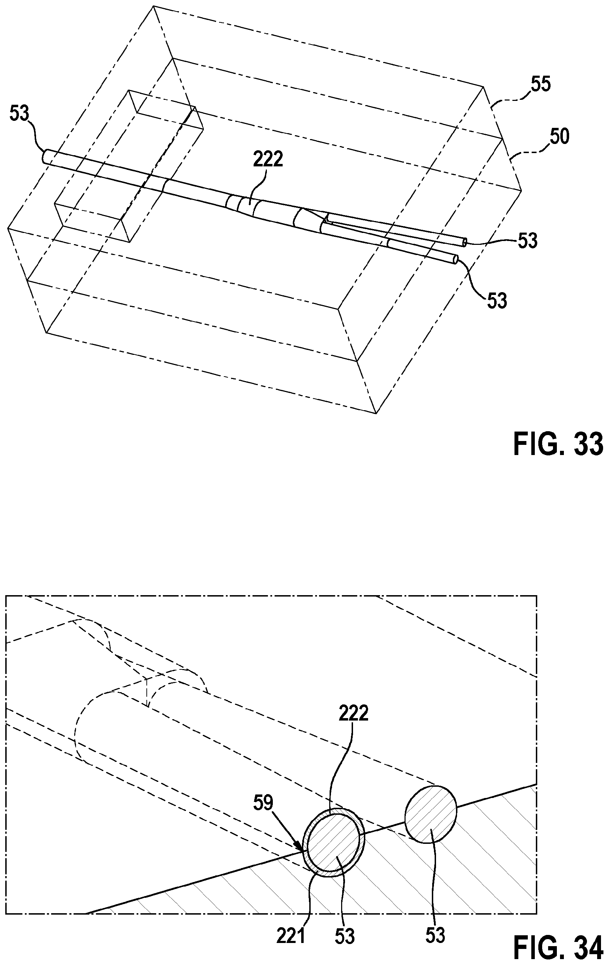

Alternatively, as shown in Fig. 29 to 34 retaining and positioning pins 53 may be used to position the first half 221 and the second half 222 correctly within a recess 51 of the carrier 50. Fig. 29 shows the carrier 50 with the recess 51 and retaining and positioning pins 53. Then, the first half 221 is inserted within the recess 51 of the carrier 50 (Fig. 30). The positioning pins 53 then move and fix the first half 221 within the recess 51 of the carrier 50 (Fig. 31, see arrows 56). Afterwards, the second half 222 is provided and positioned adjacent to the first half 221 (Fig. 32). Subsequently, the second half 222 is fixed by the lid 55 placed on top of the second half 222 thereby fixing the second half 222, as well. As shown in Fig. 34 - see arrow 59, by using the positioning and retaining pins 53 the molten material of the first half 221 and the second half 222 cannot run away during welding thereby avoiding unintended contour changes. The adjoining surfaces of the first half 221 and the second half 222 are correctly aligned.

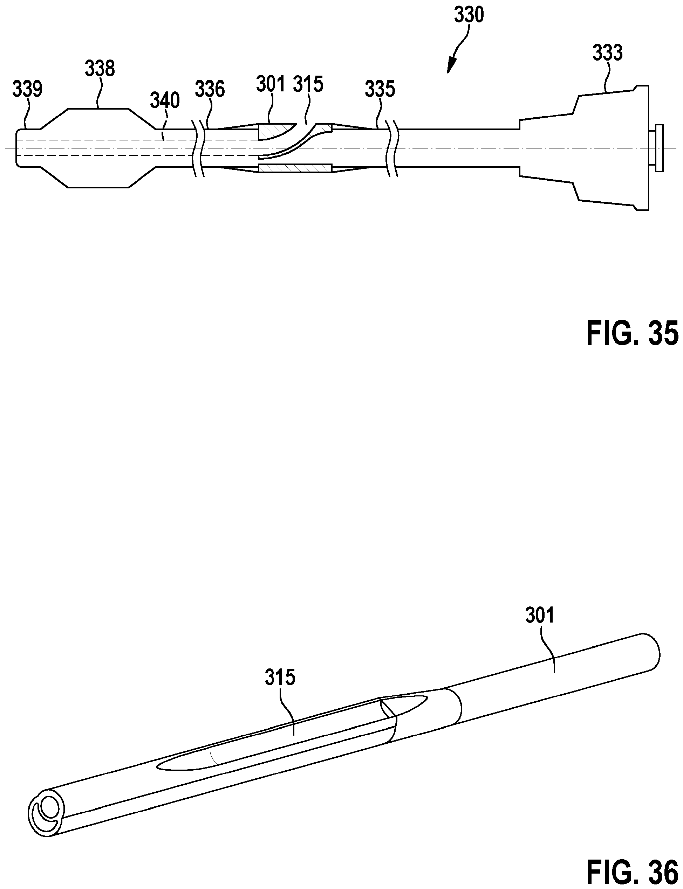

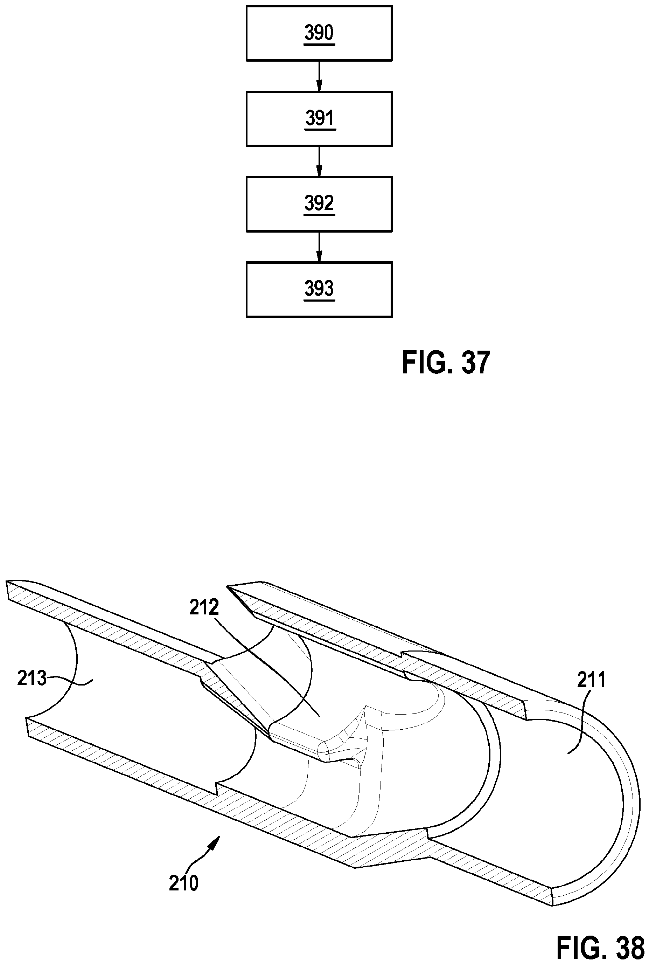

Fig. 35 shows a second embodiment of a catheter 330 comprising a handle 333 at its proximal end, a hypotube 335, an outer tubing 336 (with inner tubing), a balloon 338, a distal tip 339 and a guide wire lumen 340 shown as a double dashed line. The catheter comprises a guide wire exit port 301 with an opening 315 for introducing the guide wire. The guide wire runs outside and along the catheter 330 in the region proximal from the guide wire exit port 301. The guide wire lumen 340 mostly corresponds to the lumen within the inner tubing. An example of a guide wire exit port 301 which can be used with catheter 330 is shown in Fig. 36. This guide wire exit port 301 has a structure similar to the first, second and third embodiment of the guide wire exit port 1, 101, 201 and is manufactured as a single guide wire exit port member by additive manufacturing, for example selective laser melting, selective electron beam melting or metal binder jetting. In one example, the guide wire exit port 301 may consist of stainless steel 304. Manufacturing steps for such guide wire exit port 301 are explained with respect to the flowchart of Fig. 37 in the following. In the first step 390 the guide wire exit port 301 is manufactured, for example, by selective laser melting based on CAD data. Then, in step 391 the guide wire exit port 301 is removed from the selective laser melting facility. Subsequently, first the outer surface is post-processed, for example, by trowalizing and second the inner surface of the guide wire exit port 301, for example, by microjetting to receive smooth surfaces.

Then, the catheter may be manufactured by inserting the hypotube, the outer tubing, and the inner tubing into the proximal and the distal end of the guide wire exit port 301, respectively, by moving along the longitudinal direction and attaching them to the guide wire exit port 301, for example, by laser welding as explained with respect to Fig. 18 and 19 above.

As one can derive from the above explanation, automation of the manufacturing of a RX catheter and its guide wire exit port according to the invention can be realized thereby saving production time and costs.

Fig. 38 shows an embodiment of a first half shell 210 of a guide wire exit port. The half shell 210 has a center insert zone in a center section 212 adapted to receive an inner tubing, a distal insert zone in a distal section 211 adapted to receive an outer tubing with an inner tubing and a proximal insert zone in a proximal section 213 adapted to receive a hypotube. The guide wire exit port is manufactured, for example, such that two half shells (first half shell 210 and a matching second half shell (not shown)) are first produced separately by injection molding and/or additive manufacturing. Then, the two half shells are connected to each other. For example, the first and second half shell are accommodated such that the two shells are adjacent to one another in (close) contact and are connected using laser welding (e.g. Raman-Laser at a wavelength of 1725 nm). With additive manufacturing, e.g. 3D-printing the guidewire exit port may be manufactured as a single piece (e.g. when being cut in longitudinal direction having the shape of the half shell shown in Fig. 38).

## Claims

1. A manufacturing method for a guide wire exit port (1, 101, 201, 301), wherein the guide wire exit port is adapted to receive a hypotube (35) at a proximal end and an outer tubing (36) with an inner tubing (37) at a distal end, wherein the guide wire exit port is manufactured by additive manufacturing a single guide wire exit port member (301) or at least two guide wire exit port members and/or by injection molding at least two guide wire exit port members (21, 22, 121, 122, 221, 222), wherein the at least two guide wire exit port members are connected thereafter.

2. The method of claim 1, wherein the at least two guide wire exit port members (21, 22, 121, 122, 221, 222) are pressure-tightly connected by laser welding or fusing.

3. The method of any one of claims 1 to 2, wherein the additive manufacturing method is selective laser melting, selective electron beam melting or metal binder jetting.

4. The method of any one of claims 1 to 2, wherein the injection molding is provided using film gate (72) or pin gate (71).

5. The method of any one of claims 1 to 4, wherein the single guide wire exit port member (301) or the at least two guide wire exit port members is/are post-processed after additive manufacturing or injection molding, for example with regard to its/their outer surface and/or its/their inner surface.

6. The method of any one of claims 1 to 5, wherein the at least two guide wire exit port members are connected using at least one positioning aid (226, 227) that is provided at each one of the at least two guide wire exit port members (221, 222) of the guide wire exit port.

7. A guide wire exit port (1, 101, 201, 301) manufactured using the manufacturing steps defined in any one of the claims 1 to 6.

8. The guide wire exit port of claim 7, wherein the guide wire exit port comprises a distal section (11, 111, 211), a proximal section (13, 113, 213) and a center section (12, 112, 212) located between the distal section and the proximal section, wherein the center section comprises the opening (15, 115, 215) for the guide wire, wherein the center section has a wall thickness (w12) that is at least 30% greater than a wall thickness (w11) of the distal section and/or a wall thickness (w13) of the proximal section.

9. The guide wire exit port of any one of the claims 7 to 8, wherein the distal section has an outer diameter (D11) that is between 120% and 150% of an outer diameter (D13) of the proximal section.

10. The guide wire exit port of any one of the claims 7 to 9, wherein the center section (12, 112) comprises a web (17, 117) that extends longitudinally from the outer wall of the proximal section into the center section and divides the inner lumen of the center section into two compartments (18, 19, 118, 119) along a portion of the center section having a length (L17) in longitudinal direction of at least 1.5 mm.

11. A catheter (30, 330) comprising a guide wire exit port (1, 101, 201, 301) of one of the claims 7 to 10.

12. A manufacturing method for a catheter (30, 330), wherein the method comprises:
- providing a hypotube (35), an outer tubing (36) with an inner tubing (37) and a guide wire exit port (1, 101, 201, 301) or at least two guide wire exit port members (21, 22, 121, 122, 221, 222) of a guide wire exit port, wherein the guide wire exit port is manufactured using the method steps defined in any one of the claims 1 to 6, and
- attaching the hypotube, the outer tubing and the inner tubing to the guide wire exit port or to one of its members.

13. The method of claim 12, wherein the method comprises:
- inserting the hypotube (35) into a proximal section (113, 213) of the guide wire exit port (101) or of one of its members (121, 221),
- inserting the outer tubing (36) and the inner tubing (37) into a distal section (111, 211) of the guide wire exit port (101) or of one of its members (121, 221),
wherein in case the guide wire exit port is manufactured from at least two guide wire exit port members the hypotube and/or the inner tubing and/or the outer tubing is/are inserted into the guide wire exit port after the at least two guide wire exit port members of the guide wire exit port are pressure-tightly connected or into one of at least two guide wire exit port members of the guide wire exit port prior the at least two guide wire exit port members are pressure-tightly connected.

14. The method of any one of the claims 12 to 13, wherein attaching the hypotube (35), the inner tubing (37) and/or the outer tubing (36) is provided by additive manufacturing or injection molding and/or wherein attaching the hypotube, the outer tubing and/or the inner tubing to the guide wire exit port (201) or to one of its members is provided by laser welding or by fusing.

15. A catheter (30, 330) comprising a hypotube (35), an outer tubing (36) with an inner tubing (37) and a guide wire exit port (1, 101, 201, 301), wherein the catheter was manufactured using the manufacturing steps defined in any one of the claims 12 to 14.
